(19) **Europäisches Patentamt / European Patent Office / Office européen des brevets**

(11) **EP 2 710 951 A1**

(12) # EUROPEAN PATENT APPLICATION
published in accordance with Art. 153(4) EPC

(43) Date of publication:
**26.03.2014 Bulletin 2014/13**

(21) Application number: **12786165.6**

(22) Date of filing: **21.05.2012**

(51) Int Cl.:
*A61B 5/00* (2006.01)   *G06F 19/00* (2011.01)

(86) International application number:
**PCT/ES2012/070358**

(87) International publication number:
**WO 2012/156568 (22.11.2012 Gazette 2012/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2011 ES 201130811 U**

(71) Applicant: **Universidad Politécnica de Valencia 46022 Valencia (ES)**

(72) Inventors:
- **BONDÍA COMPANY, Jorge**
  **E-46022 Valencia (ES)**
- **BARCELÓ RICO, Fátima**
  **E-46022 Valencia (ES)**

- **DÍEZ RUANO, José Luís**
  **E-46022 Valencia (ES)**
- **ROSSETTI, Paolo**
  **E-46022 Valencia (ES)**
- **VEHI CASELLAS, Josep**
  **E-17071 Girona (ES)**
- **LEAL MONCADA, Yenny Teresa**
  **E-17071 Girona (ES)**

(74) Representative: **Pons Ariño, Angel**
**Pons Patentes y Marcas Internacional, S.L.**
**Glorieta Rubén Dario 4**
**28010 Madrid (ES)**

(54)   **SYSTEM AND METHOD FOR ESTIMATING GLUCOSE IN PLASMA**

(57)   The invention relates to a system for estimating glucose in plasma, comprising: a sensor that generates a signal from a concentration of glucose measured in a medium, filtering means, and a glycemic estimator. The invention also relates to a method comprising the following steps consisting in: i) generating a signal that represents the concentration of glucose measured in the medium; ii) filtering the signal generated; iii) applying a set of local estimation models to the aforementioned signal, thereby obtaining a set of local glucose-in-plasma estimations; iv) applying a weighting to each of the local estimations obtained; v) estimating a concentration of glucose in plasma by adding together the weighted local estimations obtained in the previous step; and vi) correcting the signal obtained in the preceding step using blood sugar reference measurements, and obtaining the final glucose-in-plasma estimation.

FIG. 1

**Description**

<u>OBJECT OF THE INVENTION</u>

The present invention, as expressed in the heading of this specification, relates to a system and to a plasma glucose estimation method the object of which is to provide a system and a continuous glucose monitoring method the accuracy of results whereof represents a substantial advance with respect to the systems and methods of the state of the art.

The field of application of the present invention is the continuous monitoring of glucose in diabetes and other states of altered glycosidic metabolism (e.g. critical patients). Within this field we can highlight: real-time monitoring, retrospective monitoring, monitoring for control of insulin infusion in current therapies, monitoring in semi-closed loop and closed loop systems in combination with insulin pump and control algorithm (artificial pancreas) and monitoring with minimally invasive and non-invasive sensors. Another field of application of the present invention is the continuous monitoring of other analytes measured in compartments alternative to the "gold standard" and which, therefore, require a form (system and/or method) of estimation (e.g. oxygen saturation in hemoglobin).

**BACKGROUND OF THE INVENTION**

The monitoring of capillary glycemia is a pillar of the treatment of diabetic patients. In recent years, the continuous monitoring of glucose in subcutaneous tissue has been disseminated as an additional tool to improve glycemic control. This type of monitoring, called minimally invasive, is based on the measurement of the glucose concentration in interstitial fluid, i.e. in a compartment other than that traditionally used as reference (the plasma). Non-invasive monitoring technologies are also being developed which are analysed in a determined glucose concentration in determined properties or signals. The analysis of these variations enables estimating the plasma glucose concentration ("glycemia"). The performance of these monitors is still very poor in comparison with the minimally invasive ones, so that the regulatory agencies have not yet allowed their marketing.

The direct or indirect measurement of glucose concentration in compartments alternative to plasma involves the need for plasma glucose estimation methods, which is the variable of interest. These methods are known as "calibration methods", contributing to the accuracy of the estimate. Said accuracy is commonly measured by the mean absolute relative difference (MARD) with respect to measurements in venous, arterial or capillary blood ("gold standard"). The following table shows some continuous monitors currently on the market, all minimally invasive, together with the reported accuracy.

| Continuous monitor | MARD (mean/median) | Reference glucose | Source |
|---|---|---|---|
| Abbott Freestyle Navigator | 12.8%/9.3% | Venous (YSI) | Weinstein et al. (2007) |
| Medtronic Guardian REAL-time | 19.9%/16.7% | Venous (YSI) | Mazze et al. (2009) |
| Medtronic Paradigm VEO | 15.89%/11.56% | Capillary (glucometer) | Keenan et al. (2010) |
| DexCom SEVEN | 16.7%/13.2% | Venous (YSI) | Zisser et al. (2009) |
| SexCom SEVEN Plus | 15.9%/13% | Venous (YSI) | Bailey et al. (2009) |

The drawbacks presented by the systems and methods of the state of the art are relative to the fact that the calibration methods of the previously mentioned monitors are based on linear regression techniques, with the consideration of a pure delay between interstitial and plasma glucose. Therefore, the information on the dynamic between the different biological compartments is ignored and so high errors can consequently be justified, at least partially. This is demonstrated in Facchinetti et al. JDST, 1 (5), 2007 by recalibration and deconvolution techniques of a dynamic plasma-interstitial model (not applicable in real time). In Facchinetti et al., DTT, 12(5), 2010 an extended Kalman filter is used in series with the standard calibration algorithm of the continuous monitor for the real-time consideration of the dynamic and improvement of precision, even though results are presented in simulation that cannot be extrapolated to clinical use. Leal et al., JDST, 4(2), 2010, demonstrates, although in a limited study, how the consideration of the dynamic behaviour expressed by a self-regressive model allows the accuracy to be improved, achieving a MARD of 9.6% (mean) / 6.7% (median). However, said accuracy worsens in the hypoglycemic range with errors of 8.1 mg/dL (mean) and 6.0 mg/dL (median) and an ISO value of 86.7%. This is due to the fact that the plasma-interstitial relation is complex and possibly dependent on the patient's metabolic state, so that the description of said relation by a single model (of global scope) may be insufficient.

It is precisely the magnitude of the error of the current systems which have conditioned approval by the regulatory agencies of the continuous monitoring of glucose only as a complementary, and not substitutive, tool, to capillary measurement. Additionally, the inaccuracy of the continuous monitoring of glucose is nowadays the greatest limiting factor in the development of artificial pancreas for the automation of glycemic control in patients with diabetes. The importance of improving the accuracy of continuous monitors is evident in the requirements of the ICT call 4 of FP7, where the objective of reducing the MARD below 5% is suggested.

No system or technique for continuous monitoring of glucose with the accuracy with which it is performed in the present invention is known in the state of the art.

## DESCRIPTION OF THE INVENTION

Continuous monitors, both those that are minimally invasive and non-invasive, provide an indirect measurement of plasma glucose concentration. The minimally invasive monitors currently on the market are based on electrochemical sensors based on enzymatic reactions (glucose oxidase) which provide an electric signal (current intensity) representative of glucose concentration in the interstitial fluid, which is later filtered and processed by the calibration system to estimate plasma glucose. In the case of non-invasive monitors, the signal from the sensor may be of another nature, as well as the compartment directly measured. The present invention has, therefore, the objective of transforming the sensor output (possibly filtered) into estimates as exact as possible of the plasma glucose.

To achieve the objectives and avoid the drawbacks indicated in the previous paragraphs, the invention consists of a plasma glucose estimation system comprising: a sensor that generates a monitoring signal selected from a current intensity signal and an alternative signal from a glucose concentration measured in a medium selected from an interstitial fluid and a remote compartment wherefrom it is possible to obtain a signal equivalent to said current intensity with its corresponding adapted sensor, filtering means that filter the monitoring signal and a glycemic estimator. The glycemic estimator, in turn, comprises at least: two local estimation modules (static or dynamic), where each local estimation module calculates a local plasma glucose estimate from reference glycemia measurements (capillary, arterial, venous or any combination thereof) and from the monitoring signal generated by the sensor previously filtered by the filtering means; a local weighting module for each local estimation module connected in series, where each local weighting module weights the local plasma glucose estimate calculated by the local estimation module whereto it is connected; and, an adder comprising one input for each local weighting module connected to each output of each local weighting module. Such that the glycemic estimator calculates an overall estimate of plasma glucose resulting from the sum of the weightings carried out by the local weighting modules.

The glycemic estimator of the present invention further comprises a corrector module comprising at least two inputs and an output. One of the inputs of the corrector module is connected to the output of the adder, and the other input receives reference glycemia measurements, selected from capillary, arterial, venous and any combination of the previous, to, in accordance with the values of the reference measurement signals and the sum of the plasma glucose estimates calculated and weighted locally by the local estimation modules and by the local weighting modules, respectively, correct the plasma glucose estimate. The reference measurement signals serve as calibration points.

On the other hand, for the preferred embodiment, each one of the at least two local estimation modules comprises processing means of a local estimation model, which is selected from a static local estimation model and a dynamic local estimation model. In turn, the static local estimation model is selected from an individual static local estimation model and a population static local estimation model. Whilst the dynamic local estimation model is selected from an individual dynamic local estimation model and a population dynamic local estimation model.

For other embodiments of the invention, the present invention comprises a number of local estimation modules which varies between three and ten.

For all the embodiments of the invention, each local weighting module in series with each local estimation module, comprises weighting processing means based on weighting algorithms.

The adder comprising one input for each local weighting module, and an output, which is connected to an input of the corrector module, carries out the sum of the plasma glucoses calculated and weighted locally by the local estimation modules and by the local weighting modules, respectively.

Furthermore, the glycemic estimator of the present invention further comprises, for another embodiment, additional input signals. These additional input signals may be of any type, being of special relevance those which define the metabolic state of a patient, so that said additional inputs induce different dynamic behaviours between the remote and plasma compartments. The remote compartment is selected from non-invasive and invasive or also called interstitial. The additional input signals are selected from: a) binary signals: high/low insulinemia, state of hypoglycemia/non-hypoglycemia, state of hyperglycemia/non-hyperglycemia, postprandial/non-postprandial state, and others; b) continuous signals: insulinemia estimated by pharmacokinetic insulin models from information from an insulin infusion pump, and other means; c) external signals from external sensors (for example, physical activity, cardiac variability, etc.); d) external signals which evaluate the patient's state; and e) any combination of the previous.

In another embodiment, the glycemic estimator further comprises a normalization module of the filtered signal which normalizes the filtered signal, a normalization module of the additional inputs which normalizes the additional inputs, a normalization module of the reference glycemia measurements (capillary, arterial or venous) which normalizes the reference glycemia measurements and a denormalization module which denormalizes the corrector module output. Said normalization/denormalization modules comprise normalization/denormalization parameters whose adjustment allows the correction of the plasma glucose estimate. Said modules include normalization and denormalization processing means, selected from population processing means and processing means individualized for the patient by an adaptation module.

Furthermore, the glycemic estimator of the present invention further comprises, for the preferred embodiment, an adaptive filter connected to the normalization module of the filtered signal, the normalization module of the additional inputs, the normalization module of the reference glycemia measurements and the denormalization module to adjust to the patient, in real time, the plasma glucose estimate by the adjustment of the normalization/denormalization parameters. In another embodiments, it is also possible to adjust the parameters of the local estimation models, static or dynamic, and the local weighting factors (validity domino of each local model).

The glycemic estimator calculates a plasma glucose estimate (EGP) according to the following expression:

$$EGP = \sum_{i}^{c} ELMP_i = ELMP_1 + ELMP_2 + \ldots ELMP_c$$

where the variables are:

EGP: plasma glucose estimate,
$ELMP_i$ = plasma glucose estimate given by the weighted i-nth local estimation model,
c = number of local estimation models (static or dynamic). The plasma glucose estimate given by the weighted nth local estimation model is of the form:

$$ELMP_i = V_i \times ELM_i$$

where:

$V_i$: is the validity of the i-nth local estimation model (weighting factor)
$ELM_i$: the plasma glucose estimate given by the i-nth local estimation model.

The local estimation models may be static or dynamic. The static local models obtain the plasma glucose estimate from the current value of the input signals to the model (static function). Said function may be linear, affine, polynomial or other types of non-linear functions.

The dynamic local estimation models obtain the plasma glucose estimate from the current and/or past value of the input signals to the model and from past plasma glucose estimate values (i.e. they use historical information). The dynamic local estimation models are discrete models of any structure (linear, affine, polynomial, other non-linear structures), with a sampling period T. In general terms, two main characteristics have been sought for all the embodiments of the present invention. The first is that the resulting model is interpretable, both mathematically and physiologically. The second characteristic is that the local estimation models found have meaning in themselves, i.e. they are independent.

Therefore, in the preferred embodiment the following is considered:

1.- The local estimation models are dynamic with affine structure, of the form

$$ELM_i(x_{ki}\beta_i) = \beta_{i1} \cdot x_{k1} + \beta_{i2} \cdot x_{k2} + \ldots + \beta_{id} \cdot x_{kd} + \beta_0$$

where $x_k$ corresponds to the input data vector (which may contain current and past values of the signal from the sensor and additional input signals, as well as past plasma glucose estimates and $\beta_i$ corresponds to the regression parameters vector.

2.- The weighting factor $V_i$ which determines, for the current input, the validity of each one of the models in a range [0,1], is defined by a Gaussian-type function of the form:

$$V_i := \mu_i(x_k) = \prod_{j=1}^{d} e^{\left(-\frac{(x_{kj} - p_{ij})^2}{2\sigma_{ij}^2}\right)}$$

where $X_k$ represents the current value of the input vector, $x_{kj}$ are each one of its components and $p_{ij}$ and $o_{ij}$ the mean and standard deviation, respectively, of the Gaussian function which characterizes the validity of the i-nth local model, for each dimension of the input vectors. The function $\mu_i(x_k)$ (called membership function) defines the degree of validity of the model in the input space, so that when the model is absolutely not characteristic for the current value of the input then $\mu_i(x_k)=0$ and when that local model completely defines that datum then $\mu_i(x_k)=1$.

Some embodiments include a factor H in the exponent as follows:

$$V_i := \mu_i(x_k) = \prod_{j=1}^{d} e^{-\frac{1}{2}\left(\frac{(x_{kj} - p_{ij})^2}{\sigma_{ij}^2}\right)^H}$$

which allows varying the gradient of the transit zone between 0 and 1. By increasing said coefficient, the function has a greater gradient. This achieves, if desired, that said functions are locally valid.

In the preferred embodiment no restriction is applied to the sum of the weighting factors $V_i$(possibilistic approach).

In another embodiments, the membership function $\mu_i(x_k)$ may be of another type in addition to Gaussian, such as, for example, trapezoidal, triangular, sigmoidal, etc., with their respective parameterizations.

Thus, the set of local estimation models is characterized in that it has the following parameters:

1. - the number of models to consider (c)

2. - the regression coefficients of the local estimation models ($\beta_i$ parameters). For each local estimation model there will be as many parameters as elements of the input data vector (d) plus an independent term. The number of parameters (np) that there will be shall be: np =c * (d+1).

3.- the parameters which define the membership function of the local estimation models. In the case of Gaussian functions they correspond to the means and variances. There will be one term for the mean and another for the variance for each one of input data vector elements, and for each local estimation model:

$$np=c * 2d.$$

The local estimation models require tuning. The local estimation models, together with their membership functions, are tuned from current intensity data (nA), additional inputs (where relevant) and glycemia measurements in a cohort of patients, in the case of minimally invasive monitors. In the case of non-invasive monitors, the current intensity is replaced by the output of the corresponding sensor.

To ascertain the value of the parameters that best adjust the set of local estimation models and their membership functions to the data, it is necessary to compare the glycemia estimate given by the system (EGP) with a reference glycemia value. The best set of parameters shall be the one which makes the difference between the estimated value and the reference value, for the entire set of data, as small as possible. In other words, it seeks to minimize a function of the errors between the system output (plasma glucose estimate EGP) and the real output (reference glycemia). In the preferred embodiment, two types of errors are defined: the overall error and the local errors of each local estimation model, of the form:

$$\left(\varepsilon_{glo\_k}\right)^2 = (y_k - \hat{y}_k)^2 = \left(y_k - \sum_{i=1}^{c} \mu_{ik} \cdot \hat{y}_{ik}\right)^2$$

$$\left(\varepsilon_{loc\_ik}\right)^2 = \mu_{ik} \cdot (y_k - \hat{y}_{ik})^2$$

The first makes reference to the difference between the real output and the system output, whilst the second makes reference to the difference between the output of each local estimation model (local glucose estimate) and the real output, weighted by the validity of the i-nth local estimation model. The subscript k makes reference to the k-nth datum.
The error to be minimized to find the value of the parameters that best adjust the model may be either of the two or even a combination of both. In the preferred embodiment, a cost index is defined which is the sum of two terms:

$$J = \sum_{k=1}^{n}\left(\varepsilon_{glo\_k}\right)^2 + \sum_{k=1}^{n}\sum_{i=1}^{c}\left(\varepsilon_{loc\_ik}\right)^2$$

The first term corresponds to the sum of the overall quadratic error of the model for all the data, and the second the sum of all the local quadratic errors for all the data, given by the following equations:

$$\sum_{k=1}^{n}\left(\varepsilon_{glo\_k}\right)^2 = \sum_{k=1}^{n}(y_k - \sum_{i=1}^{c}\mu_{ik} \cdot ELM_i(x_k; \beta_i))^2 =$$

$$\sum_{k=1}^{n}\left(y_k - \sum_{i=1}^{c}\left(\prod_{j=1}^{d} e^{-\frac{1}{2}\left(\frac{\left(x_{kj}-p_{ij}\right)^2}{\sigma_{ij}^2}\right)^{H}} \right) \cdot ELM_i(x_k; \beta_i)\right)^2 ;$$

$$\sum_{k=1}^{n}\sum_{i=1}^{c}\left(\varepsilon_{loc\_ik}\right)^2 = \sum_{k=1}^{n}\sum_{i=1}^{c}\mu_{ik} \cdot (y_k - ELM_i(x_k; \beta_i))^2$$

$$= \sum_{k=1}^{n}\sum_{i=1}^{c}\left(\prod_{j=1}^{d} e^{-\frac{1}{2}\left(\frac{\left(x_{kj}-p_{ij}\right)^2}{\sigma_{ij}^2}\right)^{H}} \right) \cdot (y_k - ELM_i(x_k; \beta_i))^2$$

Both terms include the model parameters. The result of the optimization of the cost index J shall be the values of the parameters of the model which make the proposed structure better adjust to the system output, with the inputs considered.
Additionally, the preferred embodiment includes a weight to give more or less importance to each one of these terms. The adjustment of these weights shall be performed according to the characteristics one desires to obtain in the resulting dynamic model. In other words, if one desires a smaller local error than overall error more weight shall be given to this term in order for it to have more influence in minimization of the index. And vice-versa. The resulting cost index is:

$$I = \sum_{k=1}^{n}\left( y_k - \sum_{i=1}^{c}\left( \prod_{j=1}^{d} e^{-\frac{1}{2}\left(\frac{(x_{kj}-p_{ij})^2}{\sigma_{ij}^2}\right)^H} \right) \cdot ELM_i(x_k; \beta_i) \right)^2$$

$$+ \varphi \sum_{k=1}^{n}\sum_{i=1}^{c}\left( \prod_{j=1}^{d} e^{-\frac{1}{2}\left(\frac{(x_{kj}-p_{ij})^2}{\sigma_{ij}^2}\right)^H} \right) \cdot (y_k - ELM_i(x_k; \beta_i))^2$$

In the preferred embodiment, the input signals are previously normalized before their inclusion in the input data vector $x_k$, in which case the EGP estimate presented previously corresponds to a normalized glycemia estimate, the denormalization of which corresponds to the system output. Both the normalization and the denormalization may be population or individualized for the patient. The normalization affects at least one of the following inputs: the reference glycemia measurements (capillary, arterial or venous), the current intensity signal generated by the sensor and the additional input signals. In the case of non-invasive monitors, the current intensity is replaced by the output of the corresponding sensor called alternative signal.

Different types of normalization can be applied, corresponding to different embodiments, with the objective of putting all the variables within the same conditions: putting all within an interval, making them all have the same statistical properties, etc., etc. The latter is applied in the preferred embodiment, normalizing the variables so that all the variables have zero mean and unit variance. Thus all variables can be placed in the same frame and also have an in-terpretable statistical distribution.

By normalizing the inputs, the plasma glucose estimate given by the system will also be normalized, denormalizing it from the mean and variance of the glycemia signal for the preferred embodiment. All the additional binary inputs, to be considered by the glycemic estimator, must have the same characteristics as the other inputs. Therefore, if a normalization is applied to the rest, this will also have to be applied to them.

Finally, the filtering means are selected from analogue filters and digital filters, where said filtering means eliminate measurement noise, erroneous measurements and measurements outside a pre-established range of the monitoring signal (current intensity or alternative signal) generated by the sensor.

Said normalization may be population or individualized for the patient. In the case of individualized for the patient, the glycemic estimator includes a real-time adjustment mechanism of the normalization/denormalization parameters by an adaptive filter. From the information provided by the calibration points (capillary, arterial or venous reference glycemia), the normalization/denormalization parameters of the input and output signals will be adjusted, respectively, to minimize the error. In the case of the preferred embodiment, the normalization/denormalization parameters correspond to the mean and variance of the input and output signals. In the preferred embodiment, the adaptation shall be performed as follows:

1) The parameters are initialized at population values or estimates a priori of the mean/variance of the input signals corresponding to the patient.
2) The calculated model is applied for the calibration algorithm and the glycemia estimate is obtained with the initialization parameters.
3) In each sampling period:

a. The error is evaluated between the estimate and the real output, considering, in some embodiments, a time window. In the preferred embodiment the measurement of the error consists of the mean relative error,
b. The parameters are adjusted according to the expression:

$$P_{k+1} = P_k - \gamma \cdot f\ (error)$$

where $P_k$ is the last value of any parameter to be tuned and $P_{k+1}$ its current value. The function f is a function of the gradient of the error and (possibly) of the hessian with respect to the normalization/denormalization parameters. The parameter γ is an adjustment parameter. In some embodiments it will vary with time. In each sampling period it is possible to perform more than one repetition of the parameter adjustment to minimize the error. In the case of other normalization/denormalization means different to the preferred embodiment, the mean and the variance of the input and output signals is replaced by the corresponding normalization/denormalization parameters.

As previously mentioned, the corrector module requires calibration points to calculate the plasma glucose estimate. The calibration points can be obtained from capillary measurements. In some embodiments, these points can be obtained from blood or plasma, arterial or venous measurements, performed with suitable apparatus. The calibration points can be taken in any situation. In other words, there is no condition on the range of glycemia, the gradient of the sensor signal or the patient's state.

In order for a calibration point to be accepted it must meet a set of conditions:

a) That the difference between the glycemia obtained in the calibration point and the algorithm estimate is less than 40mg/dL or 15%

b) In the case of gradients in the sensor signal greater than 3 mg/dL, the difference referred to in the previous section will increase to 60 mg/dL or 20%, respectively.

Once a calibration point has been obtained, this will be related with the signal provided by the sensor (current intensity or alternative signal) and the glycemia estimate given by the system (EGP). The sensor signal will be taken at the same instant of time as the calibration signal. In some embodiments this can be considered a delay between both signals.

A new calibration point shall be required at least every 8 hours. If, after 8 hours have passed, no new calibration point is received, the system will request the patient for one. Until this is entered, the system shall continue to provide an estimate of the glycemia and request a new calibration point periodically.

In some embodiments, the system incorporates detection algorithms of erroneous estimates based on classification techniques (vector support machines, etc). Said algorithms detect possible deviations in the model estimate after a learning phase. In said case a calibration point will be requested from the patient.

On the other hand, the present invention comprises, in addition to said system, a plasma glucose estimation method. The method of the present invention comprises the following steps:

i) generating a monitoring signal, selected from a current intensity signal and an alternative signal, which represents a glucose concentration measured in a medium selected from an interstitial fluid and a remote compartment;

ii) filtering the monitoring signal generated;

iii) applying a set of local estimation models, static or dynamic, to the monitoring signal previously generated and filtered, obtaining a set of local plasma glucose estimates;

iv) applying a weighting to each one of the local estimates obtained from the previous step in accordance with the validity of each local estimation model;

v) estimating a plasma glucose concentration by the sum of the weighted local estimates obtained from the previous step;

vi) correcting the signal obtained from the previous step from reference glycemia measurements, which are selected from capillary, arterial, venous and any combination thereof), obtaining the final plasma glucose estimate.

Step iii) further comprises applying the set of local estimation models to the monitoring signal generated and filtered by step i) and to at least one additional input signal.

For another embodiment, step iii) additionally and previously comprises normalizing the monitoring signal of current generated and filtered, and the at least one additional input signal (if it exists); and step v) further comprises denormalizing the plasma glucose concentration estimate.

The local estimation model defined by the present invention for the form of preferred embodiment, is dynamic with affine structure of the form:

$$ELM_i(x_{ki};\beta_i) = \beta_{i1} \cdot x_{k1} + \beta_{i2} \cdot x_{k2} + \ldots + \beta_{id} \cdot x_{kd} + \beta_0$$

where $x_k$ is the input data vector (which may have current and past values of the signal from the sensor and additional input signals, as well as past estimates of plasma glucose) and $\beta_i$ is the regression parameters vector.

Whilst the weighting model which is applied to the local estimation model in the preferred embodiment is of the form:

$$ELMP_i = V_i \times ELM_i$$

where:

where $ELM_i$ is the plasma glucose estimate given by the i-nth local model and $V_i$ is the weighting factor so that:

$$V_i := \mu_i(x_k) = \prod_{j=1}^{d} e^{-\frac{1}{2}\left(\frac{(x_{kj}-p_{ij})^2}{\sigma_{ij}}\right)^H}$$

where $x_k$ represents the current value of the input vector, $x_{kj}$ are each one of its components and $p_{ij}$ and $o_{ij}$ the mean and standard deviation, respectively, of the Gaussian function corresponding to the i-nth local model, for each dimension of the input vectors. The function $\mu_i(x_k)$ (called membership function) defines the degree of validity of the model in the input space, so that when the model is absolutely not characteristic for the current value of the input then $\mu_i(x_k)=0$ and when that local model completely defines that datum then $\mu_i(x_k)=1$.

In another embodiments, the local model may be static or dynamic with another structure different to that of the affine (linear, polynomial, other non-lineal structures), and the weighting factor different to those previously described (trapezoidal, sigmoidal, etc.).

Finally, a series of advantages are listed that the present invention comprises compared with other systems/methods of the state of the art.

The use of local models, each one with its specific validity region, enables modelling the existing relation between plasma (the reference compartment) and alternative measurement compartments. The dynamics of glucose between different biological compartments is complex and with this methodology it is possible to describe with two or more sets of simple models (affine in the preferred embodiment) which characterize local dynamics, for example, in different metabolic states of the patient. The consideration of local dynamics of glucose between compartments is a novelty compared with the calibration algorithms currently used, and enables improving the accuracy of continuous glucose monitors. The efficacy of this methodology must be demonstrated with the analysis of a database from an EU-clamp study-, hypo- and hyperglycemic (Rossetti et al, 2006) performed in the University of Perugia, Italy, where it compared the accuracy of a continuous glucose monitor based on the microdialysis principle (Glucoday, Menarini Diagnostics, Florence, Italy) with venous glycemia measurements obtained with a reference system (Beckman Glucose analyzer II, Brea, CA);

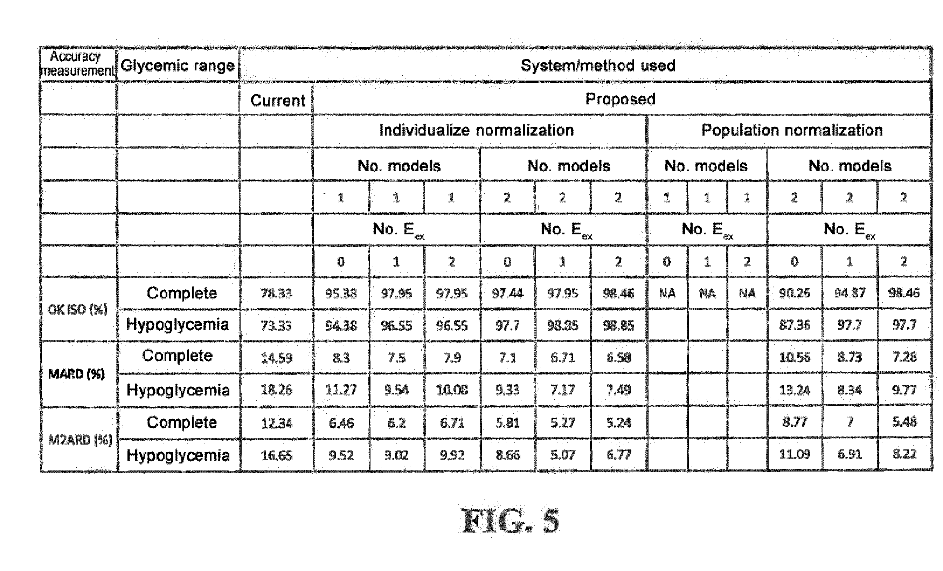

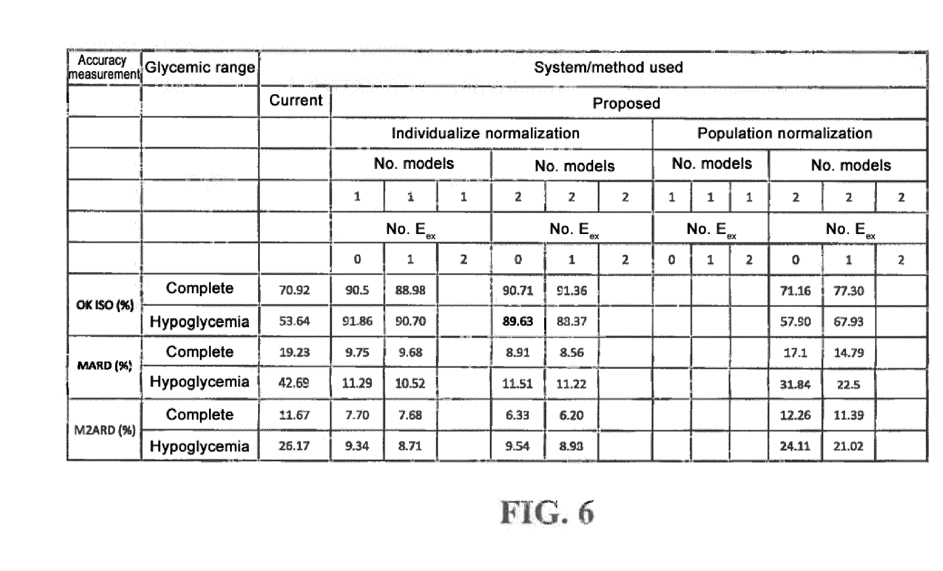

Figures 5 and 6 show the results of these validation studies. In each one of the tables represented in said figures, the accuracy obtained with the system used by the manufacturer (current system) of the continuous monitors, is compared with different system versions proposed by the present invention. The latter are differentiated, as previously indicated, by the type of normalization of the inputs (population or individual), by the number of local models c that composed the system/method, and by the number of exogenous inputs Eex that are considered. The following have been used as accuracy measurement: 1) The percentage of measurements that comply with ISO criteria (overall error <15 mg/dl with respect to the reference system, for glycemia levels < 75 mg/dl; overall error < 20% in all other cases); 2) the MARD and 3) the M2ARD (Mean and Median Absolute Relative Difference, 2) and 3) respectively).

**BRIEF DESCRIPTION OF THE FIGURES**

Figure 1.- Is a block diagram representing the system of the present invention.
Figure 2.- Is a block diagram representing the system of the present invention and where it details the glycemic estimator in the embodiment without adaptive filter.
Figure 3.- Is a block diagram representing the system of the present invention and where it details the glycemic estimator in the embodiment with adaptive filter.
Figure 4.- Is a flow diagram which represents the method of the present invention.
Figure 5.- Is a comparative table between the results obtained by the present invention against the state of the art.

Figure 6.- Is other comparative table between the results obtained by the present invention against the state of the art.

**DESCRIPTION OF ONE OR SEVERAL EMBODIMENTS OF THE INVENTION**

A description will be made below of one or several embodiments of the invention, making reference to the symbols used in figures.

First, a list is provided with the references and the elements they refer to.

| | |
|---|---|
| 1: | glycemic estimator. |
| 2: | sensor. |
| 3: | filtering means. |
| 4: | medium: interstitial fluid or remote compartment. |
| 5: | plasma glucose estimate. |
| 6: | monitoring signal generated by the sensor: current intensity signal or alternative signal. |
| 7: | filtered signal. |
| 8: | additional input signals. |
| 9: | reference glycemia measurements (capillary, arterial or venous). |
| 10: | corrector module. |
| 11: | local estimation module. |
| 11A: | local estimation module 1. |
| 11B: | local estimation module 2. |
| 11C: | local estimation module C. |
| 12: | weighting module. |
| 12A: | weighting module 1. |
| 12B: | weighting module 2. |
| 12C: | weighting module C. |
| 13: | adder. |
| 14: | processing means of a local estimation model, static or dynamic. |
| 15: | processing means of a weighting model. |
| 16: | adaptive filter. |
| 17A: | normalization module of the filtered signal. |
| 17B: | normalization module of the additional inputs. |
| 17C: | normalization module of the reference glycemia measurements. |
| 18: | denormalization module. |
| 19: | Not used. |
| 20-25: | steps of the method. |

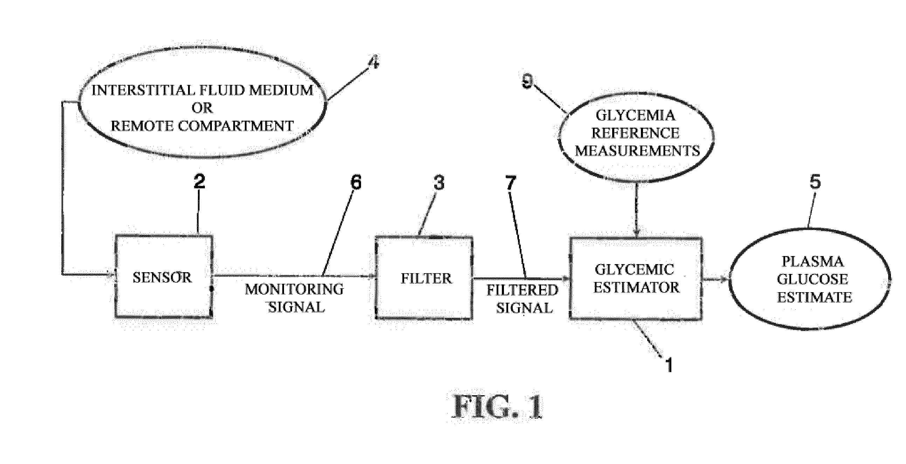

As can be observed in any of figures 1 to 4, the present invention starts from a medium 4 selected from an interstitial fluid and a remote compartment to generate a plasma glucose estimate 5. In particular, figure 1 diagrammatically represents the block diagram of the present invention so that the sensor 2 generates a monitoring signal 6 consisting of a current intensity signal or any other type of alternative signal that a sensor may generate, where the monitoring signal is representative of the glucose measured in the interstitial fluid 4 or remote compartment 4. The monitoring signal reaches a filter 3 to be filtered 7 through analogue or digital filters. Then, the filtered signal 7 reaches the glycemic estimator 1, which contains means for estimating the plasma glucose 5 from the filtered signal 7 and of the reference glycemia measurements 9, which can be capillary, arterial or venous.

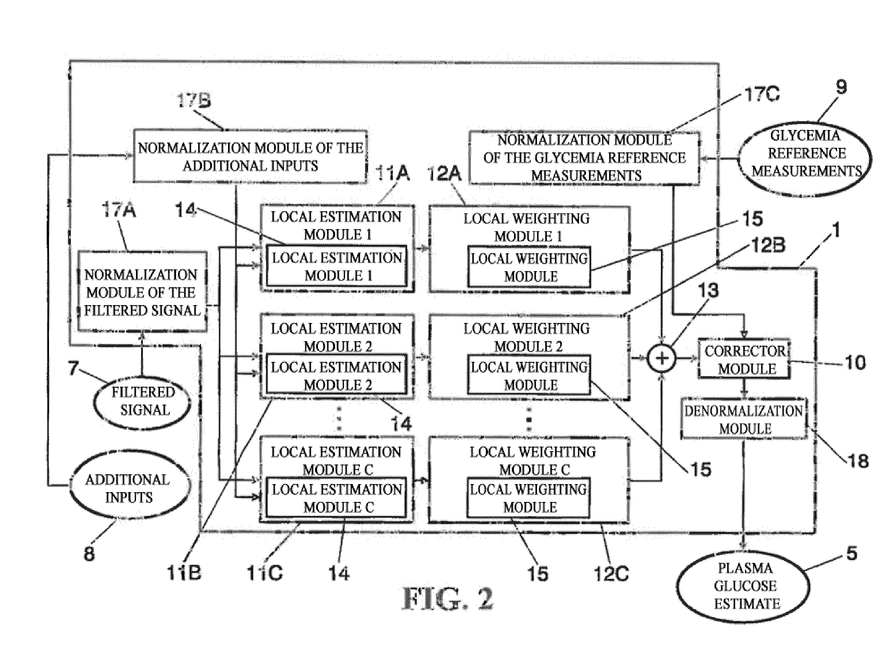

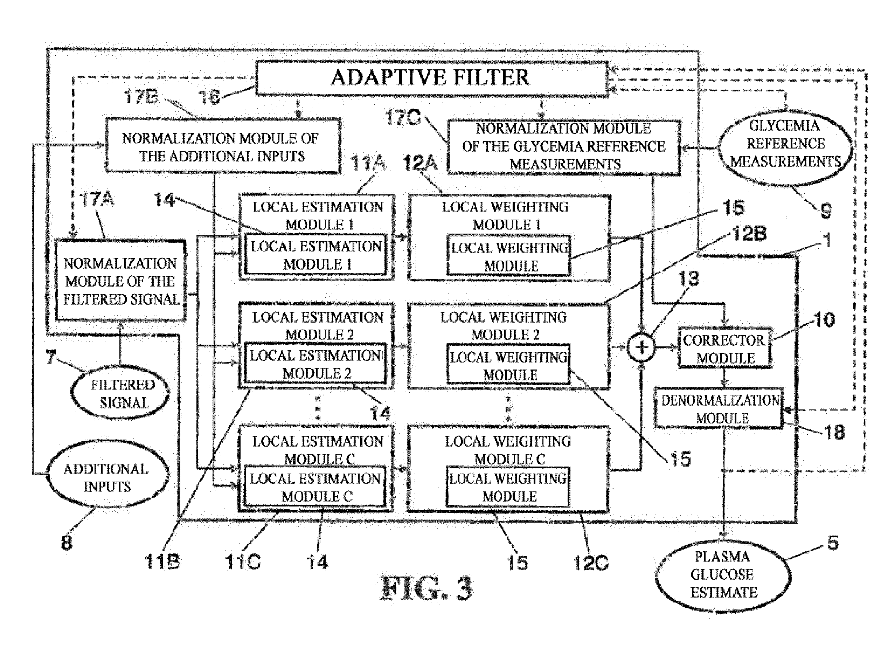

Depending on the embodiment, the glycemic estimator 1 comprises two (preferred embodiment) or several (C) local estimation models, static or dynamic (preferred embodiment) (embodiments shown in figures 2 and 3), which can be individual or population, and which express the relation between the glycemia and the sensor output.

Figure 2 shows an embodiment wherein the glycemic estimator 1 comprises several local estimates of the plasma glucose to be later weighted, added and corrected to finally generate a final estimate of the plasma glucose. In this embodiment, the glycemic estimator 1, comprises a local estimation module 1 11A, a local estimation module 2 11 B and a local estimation module C 11C, each one of which in turn comprises processing means of a local estimation model 14. The glycemic estimator 1 further comprises a local weighting module 1 12A, a local weighting module 2 12B and a local weighting module C 12C, each one of which in turn comprises processing means of a weighting model 15. Each local weighting module 12A-12C receives as input the local estimate given by the local estimation model $ELM_i$ and generates the weighted local estimate $ELMP_i$. The outputs of the local weighting modules 12A-12C are connected to the adder 13, which performs the sum of their inputs to send the result to the corrector module 10 which in accordance with the reference glycemia measurements 9, generates the plasma glucose estimate by conventional information display means, such as a display, a screen, etc. (not shown). Additionally, figure 2 shows the normalization module of the filtered signal 17A, the normalization module of the additional inputs 17B, the normalization module of the reference glycemia measurements 17C and the denormalization module 18 which normalizes the filtered signal 7, the additional inputs 8, the reference glycemia measurements 9 and denormalizes the output of the corrector module (10), respectively. The normalization may be population or individualized for the patient.

Figure 3 shows the same embodiment for the glycemic estimator 1 but where the glycemic estimator 1 further comprises an adaptive filter 16 for the adaptation in real time of the normalization/denormalization parameters in case of individualization of the patient. In this embodiment, the adaptive filter 16 adapts the normalization parameters of the additional inputs 8, the filtered signal 7 and the reference glycemia measurements 9, they all form the set of variables of input of the glycemic estimator 1. To obtain a denormalized estimate of the plasma glucose in accordance with the previously applied normalization, the adaptive filter 16 adjusts the denormalization parameters of the signal generated by the corrector module 10, so that the value shown in the display element (not shown) represents the plasma glucose estimate 5.

For the embodiments of the systems shown in figures 2 and 3, the local estimation models $ELM_i$ may be static or dynamic. The static local estimation models obtain the plasma glucose estimate from the current value of the input signals to the model (static function). Said function may be linear, affine, polynomial or other types of non-linear functions. The dynamic local estimation models obtain the plasma glucose estimate from the current and/or past value of the input signals to the model and from past plasma glucose estimate values (i.e. they use historical information). The dynamic local estimation models are discrete models of any structure (linear, affine, polynomial, other non-linear structures), with a sampling period T. Where, in the preferred embodiment, the following is considered:

1.- The local estimation models are dynamic with affine structure, of the form

$$ELM_i(x_{ki}\beta_i) = \beta_{i1} \cdot x_{k1} + \beta_{i2} \cdot x_{k2} + \ldots + \beta_{id} \cdot x_{kd} + \beta_0$$

where $x_k$ corresponds to the input data vector (which may contain current and past values of the signal from the sensor and additional input signals, as well as past plasma glucose estimates and $\beta_i$ corresponds to the regression parameters vector.

2.- The weighting factor $V_i$ which determines, for the current input, the validity of each one of the models in a range [0,1], is defined by a Gaussian-type function of the form:

$$V_i := \mu_i(x_k) = \prod_{j=1}^{d} e^{-\frac{1}{2}\left(\frac{(x_{kj}-p_{ij})^2}{\sigma_{ij}^2}\right)^H}$$

where $X_k$ represents the current value of the input vector, $x_{kj}$ are each one of its components and $p_{ij}$ and $o_{ij}$ the mean and standard deviation, respectively, of the Gaussian function corresponding to the i-nth local model, for each dimension of the input vectors. The function $\mu_i(x_k)$ (called membership function) defines the degree of validity of the model in the input space, so that when the model is absolutely not characteristic for the current value of the input then $\mu_i(x_k)=0$ and when that local model completely defines that datum then $\mu_i(x_k)=1$.

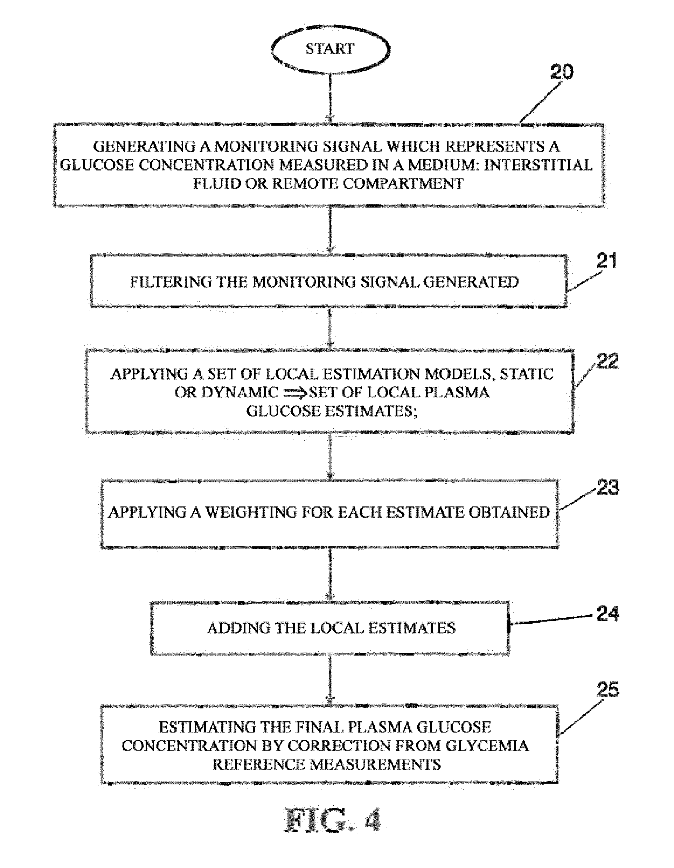

The system of the present invention carries out a method the steps whereof are represented in figure 4. Figure 4 contains six steps which correspond with the configuration of the system shown in figure 3. The six steps shown in figure 4 are the following: i) generating 20 a monitoring signal which represents the glucose concentration measured in a medium: an interstitial fluid or a remote compartment; ii) filtering 21 the monitoring signal generated; iii) applying 22 a set of local estimation models, static or dynamic, to the signal previously generated and filtered, obtaining a set of local plasma glucose estimates; iv) applying 23 a weighting model to each one of the signals obtained from the previous step; v) adding 24 the signals (local estimates) obtained in the previous step; and vi) estimating 25 the final plasma glucose concentration from the correction of the signal obtained from the previous step and from reference glycemia measurements (capillary, arterial or venous).

Finally, figures 5 and 6 show comparative tables between the results obtained by the present invention against the state of the art. The table shows a database of the clamp-Glucoday study. The performance of the system/method proposed has been assessed in its main variants: population and individual normalization; considering 1 or 2 local dynamic models; and from 0 to 2 exogenous inputs ($E_{ex}$). In particular, the following binary signals have been considered as exogenous inputs: estimated insulemia level (No. $E_{ex}=1$) or insulemia level + postprandial state (No. $E_{ex}=2$).

**Claims**

1. Plasma glucose estimation system **characterised by** comprising:

   • a sensor (2) that generates a monitoring signal (6) selected from a current intensity signal and an alternative signal from a glucose concentration measured in a medium (4) selected from an interstitial fluid and a remote compartment (4);
   • filtering means (3) that filter the monitoring signal (6); and,
   • a glycemic estimator (1) comprising at least:

      o two local estimation modules (11 A, 11B, 11C), where each local estimation module calculates a local plasma glucose estimate from reference glycemia measurements (9) and from the monitoring signal generated by the sensor (2) previously filtered through the filtering means (3);
      o a local weighting module (12, 12A, 12B, 12C) for each local estimation module connected in series,
      o where each local weighting module weights the local plasma glucose estimate calculated by the local estimation module whereto it is connected; and.
      o an adder comprising one input for each local weighting module connected to each output of each local weighting module;

   wherein the glycemic estimator calculates an overall estimate of plasma glucose resulting from the sum of the weightings carried out by the local weighting modules.

2. Plasma glucose estimation system, according to claim 1, wherein the glycemic estimator (1) further comprises a corrector module (10) of at least two inputs and an output, where said corrector module receives through one of its inputs reference glycemia measurements (9) and through the other input, the output of the adder; where the reference glycemia measurement is selected from a capillary measurement, an arterial measurement, a venous measurement and any combination thereof.

3. Plasma glucose estimation system, according to claim 1 or 2, wherein each one of the at least two local estimation modules (11A, 11B, 11C) comprises processing means of a local estimation model, which is selected from a static local estimation model and a dynamic local estimation model (14), and in turn, the static local estimation model is selected from an individual static local estimation model and a population static local estimation model, and the dynamic local estimation model is selected from an individual dynamic local estimation model individual and a population dynamic local estimation model.

4. Plasma glucose estimation system, according to any of claims 1 to 3, wherein the local weighting module (12,12A,12B,12C) comprises processing means of a weighting model (15).

5. Plasma glucose estimation system, according to any of claims 1 to 4, wherein the glycemic estimator (1) further comprises additional input signals (8).

6. Plasma glucose estimation system, according to any of claims 2 to 5, wherein the glycemic estimator (1) further comprises a normalization module of the filtered signal (17A), a normalization module of the additional inputs (17B), a normalization module of the reference glycemia measurements (17C) and a denormalization module (18) which normalizes the filtered signal (7), the additional inputs (8), the reference glycemia measurements (9) and denormalizes the output of the corrector module (10), respectively.

7. Plasma glucose estimation system, according to claim 6, wherein the glycemic estimator (1) further comprises an adaptive filter (16) connected to the normalization module of the filtered signal (17A), to the normalization module of the additional inputs (17B) and to the normalization module of the reference glycemia measurements (17C) to adjust in real time normalization parameters comprised in said normalization modules; and where said adaptive filter is additionally connected to the denormalization module (18) to adjust in real time denormalization parameters comprised in said denormalization module.

8. Plasma glucose estimation system, according to claim 5, wherein the additional input signals (8) are selected from:

a) binary signals: high/low insulinemia, state of hypoglycemia/non-hypoglycemia, state of hyperglycemia/non-hyperglycemia, postprandial/non-postprandial state; b) continuous signals of insulinemia estimated by pharmacokinetic insulin models from information from an insulin infusion pump; c) external signals from external sensors; and e) combination of the previous.

9. Plasma glucose estimation system, according to any of the preceding claims, wherein the filtering means (3) are selected from analogue filters and digital filters, where said filtering means (3) eliminate measurement noise, erroneous measurements and measurements outside a pre-established range of the monitoring signal (6) generated by the sensor (2).

10. Plasma glucose estimation method comprising:

i) generating (20) a monitoring signal selected from a current intensity signal and an alternative signal, which represents a glucose concentration measured in a medium selected from an interstitial fluid and a remote compartment;
ii) filtering (21) the monitoring signal generated;
iii) applying (22) a set of local estimation models, static or dynamic, to the monitoring signal previously generated and filtered, obtaining a set of local plasma glucose estimates;
iv) applying (23) a weighting to each one of the local estimates obtained from the previous step in accordance with the validity of each local estimation model;
v) estimating (25) a plasma glucose concentration by the sum of the weighted local estimates obtained from the previous step;
vi) correcting the signal obtained from the previous step from reference glycemia measurements, which are selected from capillary, arterial, venous and any combination thereof, obtaining the final plasma glucose estimate.

11. Plasma glucose estimation method, according to claim 10, wherein step iii) further comprises applying the set of local estimation models to the monitoring signal generated and filtered by step i) and to at least one additional input signal.

12. Plasma glucose estimation method, according to claim 11, wherein step iii) additionally and previously comprises normalizing the monitoring signal generated and filtered; step vi) additionally and previously comprises normalizing the glucose reference measurement; and step v) further comprises denormalizing the plasma glucose concentration estimate.

13. Plasma glucose estimation method, according to claim 12, wherein step iii) additionally and previously comprises normalizing the at least one additional input signal.

14. Plasma glucose estimation method, according to claim 10, wherein the local estimation model is dynamic of the form:

$$ELM_i(x_{ki}\beta_i) = \beta_{i1} \cdot x_{k1} + \beta_{i2} \cdot x_{k2} + \dots + \beta_{id} \cdot x_{kd} + \beta_0$$

where $x_k$ is the input data vector and $\beta_i$ is the regression parameters vector.

15. Plasma glucose estimation method, according to claim 14, wherein the weighting model is of the form:

$$ELMP_i = V_i \times ELM_i$$

where $ELM_i$ is the local dynamic model i, and where $V_i$ is the weighting factor such that:

$$V_i := \mu_i(x_k) = \prod_{j=1}^{d} e^{-\frac{1}{2}\left(\left(\frac{x_{kj}-p_{ij}}{\sigma_{ij}}\right)^2\right)^H}$$

where $X_k$ represents the current value of the input vector, $x_{kj}$ are each one of its components and $p_{ij}$ and $o_{ij}$ the mean and standard deviation, respectively, of the Gaussian function corresponding to the i-[nth] local model for each dimension of the input vectors, and H is a factor which defies the transit zone between the values 0 and 1.

FIG. 1

FIG. 2

FIG. 3

START

GENERATING A MONITORING SIGNAL WHICH REPRESENTS A GLUCOSE CONCENTRATION MEASURED IN A MEDIUM: INTERSTITIAL FLUID OR REMOTE COMPARTMENT — 20

FILTERING THE MONITORING SIGNAL GENERATED — 21

APPLYING A SET OF LOCAL ESTIMATION MODELS, STATIC OR DYNAMIC $\Rightarrow$ SET OF LOCAL PLASMA GLUCOSE ESTIMATES; — 22

APPLYING A WEIGHTING FOR EACH ESTIMATE OBTAINED — 23

ADDING THE LOCAL ESTIMATES — 24

ESTIMATING THE FINAL PLASMA GLUCOSE CONCENTRATION BY CORRECTION FROM GLYCEMIA REFERENCE MEASUREMENTS — 25

## FIG. 4

| Accuracy measurement | Glycemic range | System/method used | | | | | | | | | | | |
|---|---|---|---|---|---|---|---|---|---|---|---|---|---|
| | | Current | Proposed | | | | | | | | | | |
| | | | Individualize normalization | | | | | | Population normalization | | | | | |
| | | | No. models | | | No. models | | | No. models | | | No. models | | |
| | | | 1 | 1 | 1 | 2 | 2 | 2 | 1 | 1 | 1 | 2 | 2 | 2 |
| | | | No. $E_{ex}$ | | | No. $E_{ex}$ | | | No. $E_{ex}$ | | | No. $E_{ex}$ | | |
| | | | 0 | 1 | 2 | 0 | 1 | 2 | 0 | 1 | 2 | 0 | 1 | 2 |
| OK ISO (%) | Complete | 78.33 | 95.38 | 97.95 | 97.95 | 97.44 | 97.95 | 98.46 | NA | NA | NA | 90.26 | 94.87 | 98.46 |
| | Hypoglycemia | 73.33 | 94.38 | 96.55 | 96.55 | 97.7 | 98.35 | 98.85 | | | | 87.36 | 97.7 | 97.7 |
| MARD (%) | Complete | 14.59 | 8.3 | 7.5 | 7.9 | 7.1 | 6.71 | 6.58 | | | | 10.56 | 8.73 | 7.28 |
| | Hypoglycemia | 18.26 | 11.27 | 9.54 | 10.08 | 9.33 | 7.17 | 7.49 | | | | 13.24 | 8.34 | 9.77 |
| M2ARD (%) | Complete | 12.34 | 6.46 | 6.2 | 6.71 | 5.81 | 5.27 | 5.24 | | | | 8.77 | 7 | 5.48 |
| | Hypoglycemia | 16.65 | 9.52 | 9.02 | 9.92 | 8.66 | 5.07 | 6.77 | | | | 11.09 | 6.91 | 8.22 |

**FIG. 5**

| Accuracy measurement | Glycemic range | System/method used | | | | | | | | | | | | |
|---|---|---|---|---|---|---|---|---|---|---|---|---|---|---|
| | | Current | Proposed | | | | | | | | | | | |
| | | | Individualize normalization | | | | | | Population normalization | | | | | |
| | | | No. models | | | No. models | | | No. models | | | No. models | | |
| | | | 1 | 1 | 1 | 2 | 2 | 2 | 1 | 1 | 1 | 2 | 2 | 2 |
| | | | No. $E_{ex}$ | | | No. $E_{ex}$ | | | No. $E_{ex}$ | | | No. $E_{ex}$ | | |
| | | | 0 | 1 | 2 | 0 | 1 | 2 | 0 | 1 | 2 | 0 | 1 | 2 |
| OK ISO (%) | Complete | 70.92 | 90.5 | 88.98 | | 90.71 | 91.36 | | | | | 71.16 | 77.30 | |
| | Hypoglycemia | 53.64 | 91.86 | 90.70 | | **89.63** | 83.37 | | | | | 57.90 | 67.93 | |
| MARD (%) | Complete | 19.23 | 9.75 | 9.68 | | 8.91 | 8.56 | | | | | 17.1 | 14.79 | |
| | Hypoglycemia | 42.69 | 11.29 | 10.52 | | 11.51 | 11.22 | | | | | 31.84 | 22.5 | |
| M2ARD (%) | Complete | 11.67 | 7.70 | 7.68 | | 6.33 | 6.20 | | | | | 12.26 | 11.39 | |
| | Hypoglycemia | 26.17 | 9.34 | 8.71 | | 9.54 | 8.93 | | | | | 24.11 | 21.02 | |

FIG. 6

EP 2 710 951 A1

# INTERNATIONAL SEARCH REPORT

| | International application No. |
|---|---|
| | PCT/ES2012/070358 |

**A. CLASSIFICATION OF SUBJECT MATTER**

*A61B5/00* (2006.01)
*G06F19/00* (2011.01)
According to International Patent Classification (IPC) or to both national classification and IPC

**B. FIELDS SEARCHED**

Minimum documentation searched (classification system followed by classification symbols)
A61B, G06F

Documentation searched other than minimum documentation to the extent that such documents are included in the fields searched

Electronic data base consulted during the international search (name of data base and, where practicable, search terms used)

EPODOC, INVENES, WPI, NPL, INSPEC, BIOSIS, MEDLINE

**C. DOCUMENTS CONSIDERED TO BE RELEVANT**

| Category* | Citation of document, with indication, where appropriate, of the relevant passages | Relevant to claim No. |
|---|---|---|
| P,X | Barceló-Rico F. et al. "A multiple local models approach to accuracy improvement in continuous glucose monitoring", Diabetes technology & therapeutics, USA, 01.2012, vol. 14, nº 1, pages 74 – 82 | 1 - 2, 4 - 5, 8, 10, 14 - 15 |
| A | Rossetti P. et al. "Estimating plasma glucose from intersticial glucose: the issue of calibration algorithms in commercial continuous glucose monitoring devices", Sensors, Suiza, 2010, vol. 10, nº 2, pages 10936 – 10952 | 1, 10, 14 - 15 |
| A | US 2010249561 A (Patek et al.) 30.09.2010 Paragraphs 54 – 62, 73 – 155; figure 1 | 1, 7, 9, 10 |
| A | US 2011040487 A (Hovorka) 17.02.2011 Claims 40 – 71 | 1, 10 |

☒ Further documents are listed in the continuation of Box C.       ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 11/09/2012 | **(20/09/2012)** |
| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> A. Cárdenas Villar <br><br><br> Telephone No. 91 3495393 |

Form PCT/ISA/210 (second sheet) (July 2009)

## INTERNATIONAL SEARCH REPORT

| | International application No. |
|---|---|
| | PCT/ES2012/070358 |

| C (continuation). | DOCUMENTS CONSIDERED TO BE RELEVANT | |
|---|---|---|
| Category * | Citation of documents, with indication, where appropriate, of the relevant passages | Relevant to claim No. |
| A | Leal Y. et al. "Real-time glucose estimation algorithm for continuous glucose monitoring using autoregressive models", Journal of diabetes science and technology, USA, 03.2010, vol. 4, n° 2, pages 391 – 403 | 1, 10, 14 - 15 |
| A | Lee J. et al. "Improved predictive models for plasma glucose estimation from multi-linear regression analysis of exhaled volatile organic compounds", Journal of applied physiology, 07.2009, vol. 107, n° 1, pages 155 – 160 | 1, 10 |

Form PCT/ISA/210 (continuation of second sheet) (July 2009)

## INTERNATIONAL SEARCH REPORT

Information on patent family members

| International application No. |
|---|
| PCT/ES2012/070358 |

| Patent document cited in the search report | Publication date | Patent family member(s) | Publication date |
|---|---|---|---|
| US2010249561A | 30.09.2010 | EP2166946A<br>WO2008157780 | 31.03.2010<br>24.12.2008 |
| US2011040487 | 17.02.2011 | WO2009047569A | 16.04.2009 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FACCHINETTI et al.** *JDST,* 2007, vol. 1 (5
- **FACCHINETTI et al.** *DTT,* 2010, vol. 12 (5
- **LEAL et al.** *JDST,* 2010, vol. 4 (2